# EUROPEAN PATENT APPLICATION

(11) **EP 2 124 056 A1**
(43) Date of publication of application: **25.11.2009**
(21) Application number: 07860356.0
(22) Date of filing: 27.12.2007
(51) Int. Cl.: G01N 33/531

(54) **IMMUNOLOGICAL DETECTION METHOD USING AVIAN ANTIBODY**

(30) Priority: 27.12.2006 JP 2006353162
(71) Applicant: Japan Science and Technology Agency, Kawaguchi-shi, Saitama 332-0012 (JP); Hiroshima University, Higashi-Hiroshima-shi Hiroshima 739-8528 (JP); WAKUNAGA PHARMACEUTICAL CO., LTD., Osaka-shi, Osaka 532-0003 (JP)
(72) Inventor: MATSUDA, Haruo, Higashi-Hiroshima-shi Hiroshima 739-8528 (JP); KANEZAWA, Fujiko, Aki-Takata-shi Hiroshima 739-1195 (JP); KUBO, Hiroyuki, Aki-Takata-shi Hiroshima 739-1195 (JP); NISHIMICHI, Norihisa, Higashi-Hiroshima-shi Hiroshima 739-0046 (JP); AOSASA, Masayoshi, Higashi-Hiroshima-shi Hiroshima 739-0046 (JP)
(74) Representative: Vossius & Partner
(86) International application number: PCT/JP2007/075134
(87) International publication number: WO 2008/078809

(57) **Abstract**

An object of the present invention is to provide an immunological detection method using an avian antibody, comprising suppressing a non-specific reaction caused by using an avian antibody. According to the present invention, there is provided an immunological detection method comprising detecting a target substance in a sample from a mammal using an avian antibody, **characterized in that** a non-specific reaction induced by the use of the avian antibody is suppressed.

## Description

### Technical Field

The present invention relates to an immunological detection method for measuring a target substance in a sample using an avian antibody or a fragment comprising a variable region of the antibody, thereby suppressing a non-specific reaction caused by the use of the avian antibody.

### Background Art

An immunological detection method using an antibody that is specific for a target substance is widely used to measure the target substance such as hormone, microbial antigen, and inflammatory substance in a sample such as blood and urine. Most of the antibodies used in such method have traditionally been mouse monoclonal antibodies.

Although mouse monoclonal antibodies have been administered to human for the purpose of diagnosis and treatment, it is known that there is an antibody named human anti-mouse antibody (HAMA) in human blood and that a non-specific reaction is induced by HAMA in an immunological assay system using a mouse antibody (Thompson, R.J., et al. (1986) Clin. Chem. (32) 476-481, Boscato, L.M., et al. (1986) Clin. Chem. (32) 1491-1495, Zweig, M.H., et al. (1987) Clin. Chem. (33) 840-844, Primus, F.J., et al. (1988) Clin. Chem. (34) 261-264). Also, patients with autoimmune diseases such as rheumatism have an autoantibody named rheumatoid factor, which is known to similarly induce a non-specific reaction (Courtenay-Luck, N.S., (1987) Cancer Res. (47) 4520-4525). A homogeneous immunological detection method without rinsing such as the immunochromatographic method, which has rapidly spread in the recent years, is particularly susceptible to the influence of such non-specific reaction. This is posing a significant problem.

In general, there are various reasons for inducing a non-specific reaction which include the physiochemical properties of the components in the detection system, the specificity of the antibody to be used, and the presence of a substance in a sample that immunoreacts with a component in the detection system. It has been known that it is extremely difficult to suppress or eliminate such a non-specific reaction.

With the advancement of technology in recent years, a number of genetic engineering techniques including the establishment of cancer cell lines of various animals for cell fusion and phage display methods have been established, which enabled researchers to prepare monoclonal antibodies of animal species other than mouse (Smith, G.P., et al. (1985) Science (228) 1315-1317). Especially, avian is evolutionally distant from mammals. Therefore, the homology of constituent protein structures between human and avian is lower than that between human and mouse. Thus, excellent antibodies for human antigens which cannot be obtained from mice, can be obtained from avain. Such antibodies are expected to be useful as effective materials for immunological detection systems.

Avian antibodies have been considered to have high specificity, not to induce non-specific reactions which are observed with mouse monoclonal antibodies (Larsson, A., et al. (1991) Clin. Chem. (37) 411-414). There has been no report on the occurrence of non-specific reactions that are caused by the use of avian antibodies in immunological detection methods using avian antibodies in any literature.

### Summary of the Invention

The present inventors have found that non-specific reactions are induced in a detection system using an avian antibody in a sample of human blood serum or plasma during research on an avian antibody (Examples 1 to 4). It was noted that such non-specific reactions were induced more strongly and more frequently than those in a detection system using a mouse antibody (data not shown). This was a surprising finding for the inventors because avian antibodies have been considered not to induce non-specific reactions.

The present inventors also confirmed that by using an avian antibody in combination with a non-avian species antibody, it becomes possible to suppress non-specific reactions that are caused by the use of an avian antibody in an immunological detection method using an avian antibody (Examples 1 and 3). Furthermore, the inventors confirmed that by using an avian antibody in combination with F(ab')2 or scFv of an avian, it becomes possible to suppress non-specific reactions that are caused by the use of an avian antibody in an immunological detection method using an avian antibody (Examples 2 and 3). Moreover, the inventors confirmed that by using an avian antibody that does not react with a target substance in combination with avian antibodies as both an immobilized antibody and a labeled antibody, it becomes possible to suppress non-specific reactions that are caused by the use of an avian antibody in an immunological detection method using an avian antibody (Example 4).

Therefore, the prevent invention is to recognize and solve the previously unknown problem that a non-specific reaction is caused by the use of an avian antibody in an immunological detection method using an avian antibody in a human sample. More specifically, it is an object of the present invention to provide an immunological detection method using an avian antibody or a fragment comprising a variable region of the antibody, thereby suppressing a non-specific reaction.

According to the present invention, there is provided an immunological detection method for detecting a target substance using an avian antibody or a fragment comprising a variable region of the antibody, wherein the method comprises (i) detecting the target substance in a sample from a mammal using one avian antibody or a fragment comprising a variable region of the antibody and at least one antibody that does not comprise a part or all of a constant region of an avian antibody or a fragment comprising a variable region of the antibody (hereinafter, the method is referred to as "the first embodiment of the method according to the present invention").

According to the present invention, there is also provided an immunological detection method for detecting a target substance using an avian antibody or a fragment comprising a variable region of the antibody, wherein the method comprises (ii) detecting a target substance in a sample from a mammal using a first avian antibody that involves in the detection of the target substance and a second avian antibody or a fragment comprising a constant region of the antibody, wherein the second antibody does not react with a substance in the detection of the target substance (hereinafter, the method is referred to as "the second embodiment of the method according to the present invention").

When a mammalian antigen is used to immunize a mammal, it has a high homology with antigens of the immunized animal itself, making it difficult to produce antibodies. However, avian, which is evolutionally distant from mammals, is able to produce antibodies that cannot be obtained from mammals. Therefore, according to the present invention, it is advantageous in that a highly practicable immunological detection method can be constructed by utilizing the usefulness of an avian antibody or a fragment comprising a variable region of the antibody and by suppressing a non-specific reaction caused by the use of an avian antibody.

### Brief Description of the Drawings

Figure 1 shows the suppression of a non-specific reaction when an avian antibody and a non-avian species antibody are used.
Figure 2 shows the suppression of a non-specific reaction when an avian antibody and a fragment comprising a variable region of the avian antibody are used.

### Detailed Description of the Invention

As used herein, an "immunological detection method" refers to a method for detecting a target substance and the like based on an antigen-antibody reaction. The detection method can be a heterogeneous or homogeneous system.

Examples of the heterogeneous detection method include an enzyme immunoassay method (EIA) and a fluorescent immunoassay method (FIA).

Examples of the homogeneous detection method include an immunochromatography method, an agglutination method, and a flow through assay method.

As used herein, examples of "sample from a mammal" include samples from human, mouse, dog, cat, cow and the like. Preferably, the sample is from human.

Used herein, a sample can be blood, saliva, urine or the like. Preferably, the sample is blood.

Used herein, the blood can be whole blood, blood serum, blood plasma or the like. Preferably, the blood is whole blood or blood serum.

As used herein, a "non-specific reaction" refers to a reaction for which it is determined that a target substance is present even when it is not. For example, it can be caused by a reaction between an avian antibody and a substance other than the target substance in a sample. It can also be caused by physical adsorption. The notion of "suppressing a non-specific reaction" means that the non-specific reaction is reduced or eliminated, or the non-specific reaction is avoided, in the immunological detection method.

As used herein, an "avian antibody" refers to an antibody in which all the region is of an avian.

An avian antibody can be a polyclonal or monoclonal antibody. Preferably, the avian antibody is a monoclonal antibody. The avian antibody can be prepared by referring to the methods described in the literature such as Lemamy, G.J., et al. (1999) Int. J. Cancer (80) 896-902, Nakamura, N., et al. (2000) Cytotechnology (32) 191-198, and Japanese Patent Laid-Open Publication No. 2005-278633.

The avian can be chicken, goose, duck or the like.
Preferably, the avian is chicken.

As used herein, an "antibody that does not comprise a part or all of a constant region of an avian antibody or a fragment comprising a variable region of the antibody" includes, but is not limited to, an avian antibody fragment of which a part or all of a constant region has been deleted, and an avian antibody of which a part or all of a constant region has been replaced by a part or all of a constant region of a non-avian species antibody, as well as a non-avian species antibody or a fragment comprising a variable region of the antibody as long as it suppresses a non-specific reaction and allows the detection of a target substance. Examples include a fragment comprising a variable region of an avian antibody, a chimeric antibody of an avian and a non-avian species, and a non-avian species antibody or a fragment comprising a variable region of the antibody.

As used herein, a "non-avian species antibody" refers to an antibody in which all the region is of a non-avian species.

A non-avian species antibody can be either a polyclonal antibody or a monoclonal antibody. Preferably, the non-avian species antibody is a monoclonal antibody. The non-avian species antibody can be prepared by referring to the method described in the literature such as Koehler, G., et al. (1985) Nature (256) 495-497.

The non-avian species is preferably a mammal. Examples include mouse, goat, and rabbit. Preferably, the mammal is a mouse.

As used herein, a "fragment comprising a variable region of a non-avian species antibody" refers to, but is not limited to, a fragment that at least comprises a variable region of a non-avian species antibody as long as it suppresses a non-specific reaction and allows the detection of a target substance. More specifically, the fragment refers to a non-avian species antibody fragment of which a part or all of a constant region has been deleted. More specifically, examples include F(ab')2, Fab and a variable-region antibody (Fv) that do not comprise the Fc region, a single chain antibody (scFv) that only comprises the variable region, and polymers thereof. Preferably, the fragment is F(ab')2. Moreover, the fragment may comprise a protein other than the antibody.

As used herein, a "chimeric antibody of an avian and a non-avian species" refers to an avian antibody of which a part or all of a constant region has been replaced by a part or all of a constant region of a non-avian species antibody.

The chimeric antibody can be prepared by the genetic engineering linking between the avian variable region that is selected by the phage display method (Smith, G.P., et al., (1985) Science (228) 1315-1317) and the like, or the cloned variable region gene of an antibody selected by the publicly known hybridoma method (Koehler, G., et al. (1985) Nature (256) 495-497) and the constant region of the antibody of a suitable other animal species by referring to the method described in Japanese Patent Laid-Open Publication No. 2005-245337.

A non-avian species antibody to be used for the preparation of the chimeric antibody is preferably of a mammal (such as human and mouse). More preferably, the antibody is of human (more specifically, it is a chicken-human chimeric antibody).

As used herein, a "fragment comprising a variable region of an avian antibody" as in "an antibody that does not comprise a part or all of a constant region of an avian antibody or a fragment comprising a variable region of the antibody" refers to, but is not limited to, a fragment that at least comprises a variable region of an avian antibody as long as it suppresses a non-specific reaction and allows the detection of a target substance. More specifically, the fragment refers to an avian antibody fragment of which a part or all of a constant region has been deleted. More specifically, examples include F(ab')2, Fab and a variable-region antibody (Fv) that do not comprise the Fc region, a single chain antibody (scFv) that only comprises the variable region, and polymers thereof. Preferably, the fragment is scFv. Moreover, the fragment may comprise a protein other than the antibody.

The fragment comprising a variable region of a non-avian species antibody and the fragment comprising a variable region of an avian antibody can be prepared by the digestion of a non-avian or avian antibody with an enzyme (Mage, M.G., (1980) Methods Enzymol. (70) 142-150, Lamoyi, E., (1986) Methods Enzymol. (121) 652-663),or by the genetic engineering linking between the variable region of an antibody selected by the phage display method (Smith, G.P., et al., (1985) Science (228) 1315-1317) and the like, or the cloned variable region gene of an antibody selected by the publicly known hybridoma method (Koehler, G., et al. (1985) Nature (256) 495-497) and an adequate other peptide by, for example, referring to the method described in Huston, J.S., et al. (1988) Proc. Natl. Acad. Sci. U.S.A. (85) 5879-5883.

### [Detection according to the First Embodiment]

In the first embodiment of the method according to the present invention, "one avian antibody or a fragment comprising a variable region of the antibody" and "at least one antibody that does not comprise a part or all of a constant region of an avian antibody or a fragment comprising a variable region of the antibody" refer to an antibody and a fragment comprising a variable region of the antibody, wherein the antibody or a fragment can specifically recognize a target substance, a substance that specifically recognizes the target substance, and a chemical substance that modifies the substance that specifically recognizes the target substance. They are used for the purpose of detecting the target substance.

As used herein, examples of "substance that specifically recognizes the target substance" include an antibody and receptor.

As used herein, examples of "chemical substance that modifies the substance that specifically recognizes the target substance" include biotin, FITC and proteins such as peptides.

In the first embodiment of the method according to the present invention, at least one antibody or a fragment comprising a variable region of the antibody "selected from the group consisting of a non-avian species antibody or a fragment comprising a variable region of the antibody, a chimeric antibody of an avian and a non-avian species, and a fragment comprising a variable region of an avian antibody" can be a combination of two or more of any one of the antibody or a fragment comprising a variable region of the antibody in the group. For example, a combination of two mouse antibodies can be used as the antibodies of a non-avian species.

In the first embodiment of the method according to the present invention, at least one antibody or a fragment comprising a variable region of the antibody "selected from the group consisting of a non-avian species antibody or a fragment comprising a variable region of the antibody, a chimeric antibody of an avian and a non-avian species, and a fragment comprising a variable region of an avian antibody" can also be a combination of one or more of each antibody or a fragment comprising a variable region of the antibody in the group. For example, a combination of a mouse antibody as the non-avian species antibody, a chicken-human chimeric antibody as the chimeric antibody, and scFv as the fragment comprising a variable region of the avian antibody can be used.

In the first embodiment of the method according to the present invention, a combination of one avian antibody and at least one antibody that does not comprise a part or all of a constant region of an avian antibody or a fragment comprising a variable region of the antibody can be used. Alternatively, a combination of a fragment comprising a variable region of one avian antibody and at least one antibody that does not comprise a part or all of a constant region of an avian antibody or a fragment comprising a variable region of the antibody can be used. Preferably, one avian antibody is used. In such case, a "fragment comprising a variable region of one avian antibody" refers to, but is not limited to, a fragment that at least comprises a variable region of an avian antibody as long as it allows the detection of a target substance. More specifically, the fragment refers to an avian antibody fragment of which a part or all of a constant region has been deleted. More specifically, examples include F(ab')2, Fab and a variable-region antibody (Fv) that do not comprise the Fc region, a single chain antibody (scFv) that only comprises the variable region, and polymers thereof. Preferably, the fragment is scFv. Moreover, the fragment can comprise a protein other than the antibody. Furthermore, when a combination of a fragment comprising a variable region of one avian antibody and a fragment comprising a variable region of an avian antibody as at least one antibody that does not comprise a part or all of a constant region of an avian antibody or a fragment comprising a variable region of the antibody is used, the "fragment comprising a variable region of one avian antibody" and the "fragment comprising a variable region of an avian antibody" can be fragments containing the identical variable region of an avian antibody.

In the first embodiment of the method according to the present invention, regarding one avian antibody or a fragment comprising a variable region of the antibody and at least one antibody that does not comprise a part or all of a constant region of an avian antibody or a fragment comprising a variable region of the antibody to be used in (i), either one can be used as an immobilized antibody and either one can be used as a labeled antibody. Furthermore, either one or more can be used as a first antibody in the indirect method.

An immobilized antibody can be a first antibody, more specifically, an antibody against the antigen (target substance) (the direct method). An immobilized antibody can also be a second antibody, more specifically, an antibody against a substance (if the substance is an antibody, it corresponds to the first antibody) that specifically recognizes the target substance, or against a chemical substance that modifies the substance that specifically recognizes the target substance (the indirect method). The immobilized antibody can be anchored on a plate or membrane.

A labeled antibody can be a labeled first antibody, more specifically, a labeled antibody against the antigen (target substance) (the direct method). A labeled antibody can also be a labeled second antibody, more specifically, a labeled antibody against a substance (if the substance is an antibody, it corresponds to the first antibody) that specifically recognizes the target substance, or against a chemical substance that modifies the substance that specifically recognizes the target substance (the indirect method). Examples of the labeled substance include enzyme, fluorescent substance, biotin, gold colloid, latex particles.

In the first embodiment of the method according to the present invention, one can prepare the immobilized antibody and labeled antibody as the first antibodies to perform the immunological detection by the direct method.

In the first embodiment of the method according to the present invention, one can prepare the immobilized antibody as the first antibody and the labeled antibody as the second antibody, or the immobilized antibody as the second antibody and the labeled antibody as the first or second antibody to perform the immunological detection by the indirect method.

In the first embodiment of the method according to the present invention, the presence of the antigen-antibody complex serves as an index for the presence of the target substance. Therefore, when the antigen-antibody complex (such as the labeled antigen-antibody complex) is detected by the immunological detection method, one can determine that the target substance is present in the sample.

### [Detection according to the Second Embodiment]

In the second embodiment of the method according to the present invention, a "first avian antibody that involves in the detection of a target substance" refers to an avian antibody that can specifically recognize a target substance, a substance that specifically recognizes the target substance, and a chemical substance that modifies the substance that specifically recognizes the target substance. The antibody is used for the purpose of detecting the target substance. The "first avian antibody" to be used in the second embodiment of the method according to the present invention can comprise only one or two or more kinds of avian antibodies as long as it enables one to perform a sandwich method. When two or more kinds of avian antibodies are used, either one or more kinds of the avian antibodies can be used as an immobilized antibody (such as an immobilized antibody and an antibody that is recognized by the immobilized antibody) and the other one or more kinds of the avian antibodies can be used as a labeled antibody (such as a labeled antibody and an antibody that is recognized by the labeled antibody). When one kind of avian antibodies are used, the same kind of avian antibodies can be used both as an immobilized antibody and labeled antibody.

As used herein, a "second avian antibody that does not react with a substance in the detection of a target substance" refers to a competitive antibody that is not directly involved in the detection of the target substance. The antibody can be prepared by purifying the avian blood serum or egg, or by the publicly known genetic engineering method. Moreover, a "fragment comprising a constant region" of the second avian antibody refers to, but is not limited to, a fragment that at least comprises a part or all of a constant region of the second avian antibody that does not react with a substance in the detection of the target substance as long as it cab be used as a competitive antibody. The fragment is prepared by the publicly known genetic engineering method. The fragment can comprise a protein other than the antibody (hereinafter, the "second avian antibody that does not react with a substance in the detection of a target substance" and "fragment comprising a constant region" are together referred to as a "competitive antibody").

The presence of such competitive antibody in the reaction system of (ii) can effectively suppress a non-specific reaction. The competitive antibody is preferably added to the reaction system in advance, or added to the reaction system with the other antibodies.

Examples of "substance in the detection of a target substance" include the target substance; an avian antibody, a non-avian species antibody or a fragment comprising a variable region of the antibody, a chimeric antibody of an avian and a non-avian species, a fragment comprising a variable region of an avian antibody and a labeled substance, all of which are to be used in the detection of the target substance. The EIA method is used to determine whether or not the antibody reacts with the target substance. The EIA method and the like are used to determine whether or not the antibody reacts with the avian antibody, the non-avian species antibody or the fragment comprising a variable region of the antibody, the chimeric antibody of the avian and non-avian species, or the fragment comprising a variable region of the avian antibody. The EIA method and the like are used to determine whether or not the antibody reacts with the labeled substance.

In the second embodiment of the method according to the present invention, the amount of the competitive antibody to be added is preferably in the range of 1 to 50 µg per kit.

In a heterogeneous system such as EIA and FIA, the second avian antibody to be used as the competitive antibody can be added to the reaction solution containing the substance to be used for the detection of the target substance, or added to a diluted solution of a sample such as blood serum. In a homogenous system such as the immunochromatographic method, the second avian antibody can be added in the mobile or immobile state upstream of an antibody-immobilized part.

In the second embodiment of the method according to the present invention, an antibody that does not comprise a part or all of a constant region of an avian antibody or a fragment comprising a variable region of the antibody can be used in combination with the first avian antibody and competitive antibody according to need.

In the second embodiment of the method according to the present invention, regarding first avian antibodies, or in some cases, a first antibody and an antibody that does not comprise a part or all of a constant region of an avian antibody or a fragment comprising a variable region of the antibody, all of which are to be used in (ii), one antibody can be used as an immobilized antibody and another can be used as a labeled antibody. In the indirect method, one or more additional antibodies can be used as the first antibody.

An immobilized antibody can be a first antibody, more specifically, an antibody against the antigen (target substance) (the direct method). An immobilized antibody can also be a second antibody, more specifically, an antibody against a substance (if the substance is an antibody, it corresponds to the first antibody) that specifically recognizes the target substance, or against a chemical substance that modifies the substance that specifically recognizes the target substance (the indirect method). The immobilized antibody can be anchored on a plate or membrane.

A labeled antibody can be a labeled first antibody, more specifically, a labeled antibody against the antigen (target substance) (the direct method). A labeled antibody can also be a labeled second antibody, more specifically, a labeled antibody against a substance (if the substance is an antibody, it corresponds to the first antibody) that specifically recognizes the target substance, or against a chemical substance that modifies the substance that specifically recognizes the target substance (the indirect method). Examples of the labeled substance include enzyme, fluorescent substance, biotin, gold colloid, latex particles.

In the second embodiment of the method according to the present invention, one can prepare the immobilized antibody and labeled antibody as the first antibodies to perform the immunological detection by the direct method.

In the second embodiment of the method according to the present invention, one can prepare the immobilized antibody as the first antibody and the labeled antibody as the second antibody, or the immobilized antibody as the second antibody and the labeled antibody as the first or second antibody to perform the immunological detection by the indirect method.

In the second embodiment of the method according to the present invention, the presence of the antigen-antibody complex serves as an index for the presence of the target substance. Therefore, when the antigen-antibody complex (such as the labeled antigen-antibody complex) is detected by the immunological detection method, one can determine that the target substance is present in the sample.

### Example

Hereinafter, the present invention is explained in more detail with reference to the following examples. However, it is understood that the invention is not limited thereto.

### Example 1: Suppression of Non-specific Reaction in EIA Method (1)

To a commercially available microplate (MAXISORP, manufactured by NUNC), 50 µL of chicken antibody (chicken IgG, manufactured by CAPPEL Laboratories Inc.) or anti-LH mouse monoclonal antibody (LHS14) dissolved to a 0.1 "M" sodium carbonate buffer ("pH" 10) at a concentration of 1 µg/mL was added. The microplate was left to stand for 1 hour at 37°C. After rinsing three times with a rinsing solution (PBS/0.05% Tween 20), 250 µL of a 0.5% BSA/PBS solution was added thereto, and the microplate was left to stand for 1 hour at 37°C. After rinsing three times with the rinsing solution, 25 µL of 2 µg/mL biotinated chicken antibody, or biotinated mouse monoclonal antibody and 25 µL of a 5-fold dilution of human blood serum were added thereto, and the microplate was left to stand for 1 hour at 37°C. After rinsing three times with the rinsing solution, 50 µL of a 1000-fold dilution of peroxidase-labeled streptavidin (manufactured by ZYMED Laboratories Inc.) was added thereto, and the microplate was left to stand for 1 hour at 37°C. After rinsing three times with the rinsing solution, 100 µL of a substrate-chromogen solution containing ABTS (2,2'-azino-di-[3-ethyl-benzothiazoline-sulfonic acid (6)]-diammonium salt) and hydrogen peroxide was added thereto, and the microplate was left to stand for 15 min at 37°C. The absorbance at the wavelength of 415 nm was measured using a microplate reader (manufactured by BioRad Laboratories Inc.).

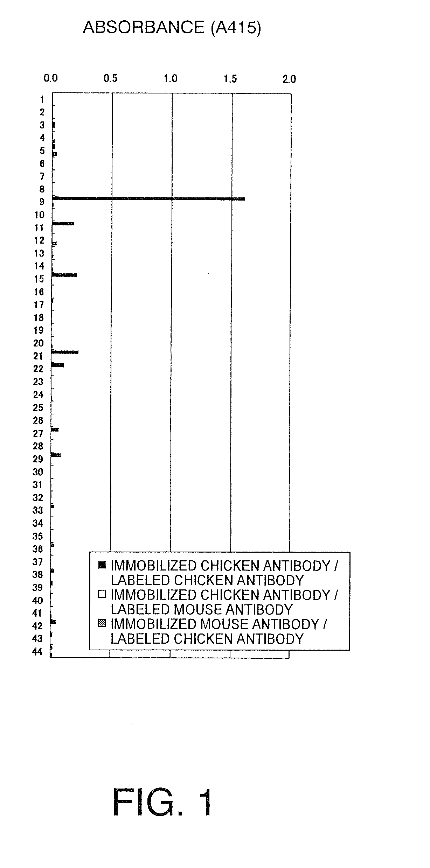

As a result, when the chicken antibody was used as both the immobilized and biotinated antibodies, multiple samples developed coloring that was suspected to indicate a non-specific reaction (Figure 1). However, it was confirmed that the non-specific reaction was significantly suppressed when the mouse monoclonal antibody was used as either the immobilized or biotinated antibody (Figure 1).

### Example 2: Suppression of Non-specific Reaction in EIA Method (2)

To a commercially available microplate (MAXISORP, manufactured by NUNC), 50 µL of chicken antibody (chicken IgG, manufactured by CAPPEL Laboratories Inc.) or single chain antibody that only comprises the variable region (scFv) dissolved to a 0.1 "M" sodium carbonate buffer ("pH" 10) at a concentration of 1 µg/mL was added. The microplate was left to stand for 1 hour at 37°C. After rinsing three times with a rinsing solution (PBS/0.05% Tween 20), 250 µL of a 0.5% BSA/PBS solution was added thereto, and the microplate was left to stand for 1 hour at 37°C. After rinsing three times with the rinsing solution, 25 µL of 2 µg/mL biotinated chicken antibody and 25 µL of a 5-fold dilution of human blood serum were added thereto, and the microplate was left to stand for 1 hour at 37°C. After rinsing three times with the rinsing solution, 50 µL of a 1000-fold dilution of peroxidase-labeled streptavidin (manufactured by ZYMED Laboratories Inc.) was added thereto, and the microplate was left to stand for 1 hour at 37°C. After rinsing three times with the rinsing solution, 100 µL of a substrate-chromogen solution containing ABTS (2,2'-azino-di-[3-ethyl-benzothiazoline-sulfonic acid (6)]-diammonium salt) and hydrogen peroxide was added thereto, and the microplate was left to stand for 15 min at 37°C. The absorbance at the wavelength of 415 nm was measured using a microplate reader (manufactured by BioRad Laboratories Inc.).

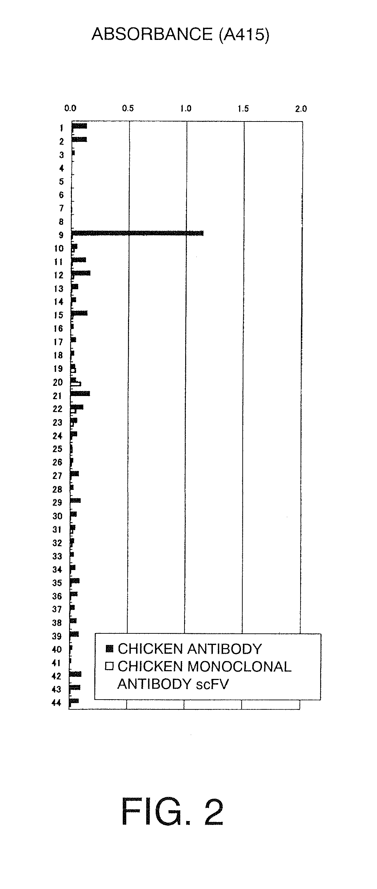

As a result, when the chicken antibody was used as both the immobilized and biotinated antibodies, coloring that was suspected to indicate a non-specific reaction was observed at a high frequency (Figure 2). However, it was confirmed that the non-specific reaction was significantly suppressed when the single chain antibody that only comprises the variable region (scFv) was used in place for the chicken antibody (Figure 2).

### Example 3: Suppression of Non-specific Reaction in Immunochromatogiraphic Method (1)

### (1) Preparation of membrane with the detection region

To a nitrocellulose membrane (Hi-Flow Plus manufactured by Millipore Corp.), chicken antibody or anti-human albumin mouse monoclonal antibody (THSA8) was applied in lines by a dispenser. After drying the membrane at 25°C, a casein solution was used for blocking and the membrane was further dried at 25°C. Finally, the membrane was cut into strips with a dimension of 5 mm x 25 mm.

### (2) Preparation of gold colloid labeled reagent

A gold colloid solution (gold-sol G40, manufactured by BBI International) was used to label the chicken antibody, mouse monoclonal antibody (THSA8), chicken monoclonal antibody F(ab')2, and single chain antibody that only comprises the variable region (scFv) according to the protocol. The gold colloid labeled reagent was applied on a conjugate pad by a dispenser, and the pad was subjected to freeze drying. Finally, the pad was cut into pieces with a dimension of 5 mm x 25 mm.

### (3) Preparation of test strip

On a polyethylene film onto which an adhesive agent was applied, the nitrocellulose membrane that had the detection region was attached. To one end, the cellulose membrane cut into a width of 5 mm x 22 mm was attached with a 3-mm overlap at the end. To the other end, a glass fiber filter paper cut into a dimension of 5 mm x 26 mm was attached with a 1-mm overlap at the end. To the other end of the glass fiber filter paper, the conjugate pad onto which the gold colloid labeled reagent was applied was attached with a 21-mm overlap.

### (4) Test

To the conjugate pad, 150 µL of a sample of human blood plasma or blood serum was added. After 15 min, the presence of a line in the detection region was checked. Ones with the line were identified as "+: presence of the non-specific reaction" and ones without the line as "-: absence of the non-specific reaction."

As a result, the combined use of the chicken antibodies was confirmed to induce the non-specific reaction at a high frequency when the chicken antibody was used as both the immobilized antibody and labeled antibody. In contrast, it was confirmed that the use of the mouse antibody either as the immobilized antibody or labeled antibody completely eliminated the occurrence of the non-specific reaction (Tables 1 and 2).

**[Table 1]**

| Immobilized antibody | Chicken antibody | Mouse antibody |
|---|---|---|
| Labeled antibody | Chicken antibody | |
| Non-specific reaction rate | 47%(21/45) | 0%(0/44) |

**[Table 2]**

| Immobilized antibody | Chicken antibody | |
|---|---|---|
| Labeled antibody | Chicken antibody | Mouse antibody |
| Non-specific reaction rate | 47%(21/45) | 0%(0/44) |

Moreover, in the system using the chicken antibody as the immobilized antibody and the chicken antibody or the fragment comprising a variable region of the chicken antibody (F(ab')2 and scFv) as the labeled antibody, it was confirmed that the non-specific reaction was induced at a high frequency when the chicken antibody was used as the labeled antibody. In contrast, it was confirmed that the use of F(ab')2 reduced the occurrence of the non-specific reaction, and that the use of scFv completely eliminated the occurrence of the non-specific reaction (Table 3).

**[Table 3]**

| Immobilized antibody | Chicken antibody | | |
|---|---|---|---|
| Labeled antibody | Chicken antibody | F(ab')2 | scFv |
| Non-specific reaction rate | 51%(24/47) | 19%(9/47) | 0%(0/47) |

### Example 4: Suppression of Non-specific Reaction in Immunochromatographic Method (2)

### (1) Preparation of membrane with the detection region

To a nitrocellulose membrane (Hi-Flow Plus manufactured by Millipore Corp.), chicken antibody was applied in lines by a dispenser. After drying the membrane at 25°C, a casein solution was used for blocking and the membrane was further dried at 25°C. Finally, the membrane was cut into strips with a dimension of 5 mm x 25 mm.

### (2) Preparation of gold colloid labeled reagent

A gold colloid solution (gold-sol G40, manufactured by BBI International) was used to label the chicken antibody according to the protocol. The gold colloid labeled reagent was applied on a conjugate pad by a dispenser, and the pad was subjected to freeze drying. Finally, the pad was cut into pieces with a dimension of 5 mm x 25 mm.

### (3) Preparation of membrane applied with competitive antibody

To a glass fiber filter paper, the chicken antibody was applied by a dispenser. After drying at 25°C, the filter paper was cut into pieces with a dimension of 5 mm x 26 mm. The amount of the chicken antibody applied to the pieces was adjusted to either 18 µg or 36 µg.

### (4) Preparation of test strip

On a polyethylene film onto which an adhesive agent was applied, the nitrocellulose membrane that had the detection region was attached. To one end, the cellulose membrane cut into a width of 5 mm x 22 mm was attached with a 3-mm overlap at the end. To the other end, a glass fiber filter paper cut into a dimension of 5 mm x 26 mm was attached with a 1-mm overlap at the end. To the other end of the glass fiber filter paper, the conjugate pad onto which the gold colloid labeled reagent was applied was attached with a 21-mm overlap.

### (5) Test

To the conjugate pad, 150 µL of a sample of human blood plasma or blood serum was added. After 15 min, the presence of a line in the detection region was checked. Ones with the line were identified as "+: presence of the non-specific reaction" and ones without the line as "-: absence of the non-specific reaction."

As a result, in the present detection system using the chicken antibody both as the immobilized antibody and labeled antibody, it was confirmed that the addition of the competitive antibody reduced and eliminated the occurrence of the non-specific reaction depending on the amount of the competitive antibody (Table 4).

**[Table 4]**

| | Without competitive antibody | Competitive antibody 18µg | Competitive antibody 36µg |
|---|---|---|---|
| Non-specific reaction rate | 51%(24/47) | 19%(9/47) | 0%(0/47) |

## Claims

1. An immunological detection method for detecting a target substance using an avian antibody or a fragment comprising a variable region of the antibody, wherein the method comprises:
(i) detecting the target substance in a sample from a mammal using one avian antibody or a fragment comprising a variable region of the antibody and at least one antibody that does not comprise a part or all of a constant region of an avian antibody or a fragment comprising a variable region of the antibody; or
(ii) detecting the target substance in a sample from a mammal using a first avian antibody that involves in the detection of the target substance and a second avian antibody or a fragment comprising a constant region of the antibody, wherein the second avian antibody does not react with a substance in the detection of the target substance.

2. The detection method according to claim 1, wherein the sample from a mammal is human blood.

3. The method according to claim 1, wherein the antibody that does not comprise a part or all of a constant region of an avian antibody or a fragment comprising a variable region of the antibody is selected from the group consisting of a non-avian species antibody or a fragment comprising a variable region of the antibody, a chimeric antibody of an avian and a non-avian species, and a fragment comprising a variable region of an avian antibody.

4. The method according to claim 3, wherein the fragment comprising a variable region of a non-avian species antibody is a non-avian species antibody of which a part or all of a constant region has been deleted.

5. The method according to claim 3, wherein the chimeric antibody is an avian antibody of which a part or all of a constant region has been replaced by a part or all of a constant region of a non-avian species antibody.

6. The method according to claim 3, wherein the fragment comprising a variable region of an avian antibody is an avian antibody of which a part or all of a constant region has been deleted.

7. The method according to any one of claims 1, 4, and 6, wherein the fragment comprising a variable region of an antibody is selected from the group consisting of F(ab')2, Fab, scFv, and Fv.

8. The method according to any one of claims 1 to 7, wherein the avian is a chicken.
